# EUROPEAN PATENT APPLICATION

(11) **EP 4 501 960 A1**
(43) Date of publication of application: **05.02.2025**
(21) Application number: 22934556.6
(22) Date of filing: 21.06.2022
(51) Int. Cl.: C07K 16/28, C12N 15/13, G01N 33/574, G01N 33/577

(54) **MONOCLONAL ANTIBODY AGAINST HLA-G MOLECULES AND USE THEREOF**

(30) Priority: 31.03.2022 CN 202210336599
(71) Applicant: Taizhou Enze Medical Center (Group), Taizhou, Zhejiang 317099 (CN)
(72) Inventor: YAN, Weihua, Linhai Taizhou, Zhejiang 317099 (CN)
(74) Representative: Kransell & Wennborg KB
(86) International application number: PCT/CN2022/100232
(87) International publication number: WO 2023/184733

(57) **Abstract**

Here provided an antibody YWHG-1 against HLA-G molecule and use thereof. The antibody is produced by the hybridoma deposited under CCTCC NO: 202120, and is prepared by an immunogen with amino acids which are common to seven known HLA-G isoforms (HLA-G1, HLA-G2, HLA-G3, HLA-G4, HLA-G5, HLA-G6 and HLA-G7) which is located at positions 72-91 (QTDRMNLQTLRGYYNQSEAS) in the heavy chain α1 domain of the HLA-G molecule. The present invention provides nucleotides encoding the antibody YWHG-1 and encoded amino acid sequences thereof. The antibody YWHG-1 can be used for immunohistochemistry, immunoblotting, flow cytometry and other assays for HLA-G isoforms.

## Description

### FIELD OF THE INVENTION

The present invention belongs to the field of biomedicine and relates to the following aspects of an antibody YWHG-1 against HLA-G molecule: a monoclonal antibody YWHG-1 against HLA-G molecule prepared by an immunogen with amino acids which are common to seven known HLA-G isoform molecules (HLA-G1, HLA-G2, HLA-G3, HLA-G4, HLA-G5, HLA-G6 and HLA-G7) and are located at positions 72-91 (QTDRMNLQTLRGYYNQSEAS) in the heavy chain α1 domain of the HLA-G molecules,; nucleotides encoding the antibody YWHG-1 and an encoded amino acid sequences thereof, and use of the antibody YWHG-1 for immunohistochemistry, immunoblotting, flow cytometry and other assays.

### BACKGROUND OF THE INVENTION

The human leukocyte antigen-G (HLA-G) gene, with a full length of 6.0 kb, is located at 6p21.3, the distal short arm of human chromosome 6. During protein translation, exon 1 of HLA-G mRNA encodes the signal peptide, exons 2, 3, and 4 encode the α1, α2, and α3 domains of the extracellular region, respectively, and exon 5 encodes the transmembrane region; exon 6 encodes the intracellular segment of HLA-G molecule containing only 6 amino acid residues. Since exon 6 contains a stop codon, exon 7 is not transcribed; exon 8 corresponds to the 3'UTR of the HLA-G gene. The initial transcript of HLA-G can be alternatively spliced to produce 7 types of mature mRNA, encoding 7 isoforms with different molecular weights (HLA-G1, -G2, -G3, -G4, -G5, -G6 and -G7). Among them, HLA-G1, HLA-G2, HLA-G3 and HLA-G4 contain transmembrane regions and are membrane-bound isoforms; HLA-G5, HLA-G6 and HLA-G7 lack transmembrane structures and are soluble isoforms. The molecular weights of HLA-G1~-G7 isoforms are 39kD, 31kD, 22kD, 30kD, 34kD, 23kD and 16kD respectively.

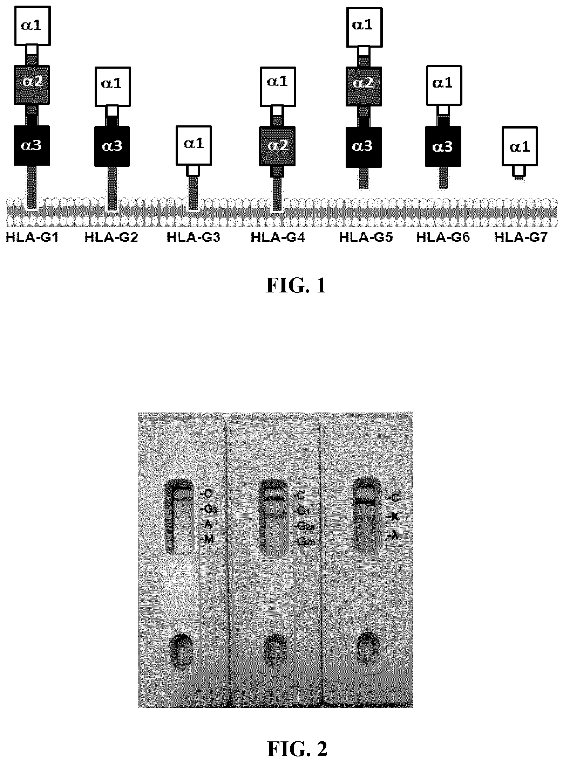

HLA-G1 is encoded by full-length HLA-G mRNA and consists of extracellular α1, α2 and α3 domains, a transmembrane region and an intracellular domain. HLA-G2 lacks α2 domain and consists of extracellular α1 and α3 domains, a transmembrane region and an intracellular domain; HLA-G3 lacks α2 and α3 domains and consists of an extracellular α1 domain, a transmembrane region and an intracellular domain; HLA-G4 lacks α3 domain and consists of extracellular α1 and α2 domains, a transmembrane region and an intracellular domain extracellular domain is the same as HLA-G1 and HLA-G2 respectively. However, they are encoded by HLA-G mRNA containing intron 4. Because intron 4 contains a stop codon, the encoded protein molecules lack the transmembrane region encoded by exon 5, forming soluble HLA-G molecules. Since the mRNA encoding HLA-G7 contains a stop codon in intron 2, the extracellular region only contains the α1 domain and is connected by the two amino acid residues encoded by intron 2 (FIG. 1).

Under normal physiological conditions, HLA-G molecule is only expressed in extravillous cytotrophoblast cells at the maternal-fetal interface, maintaining maternal-fetal immune tolerance during pregnancy. Under pathological conditions, HLA-G molecule expression can be induced in pathological tissues and cells such as virus-infected cells and tumor cells which are closely related to the occurrence and progression of the diseases. HLA-G molecule is an important immune checkpoint molecule. The immunosuppressive function of HLA-G is mainly by binding to the immunosuppressive receptors immunoglobulin-like transcript-2 (ILT2/LILRB1/CD85j) and immunoglobulin-like transcript-4 (II,T4/LII,RB2/CD85d). The underlying mechanisms of immunoinhibitory function induced by HLA-G/ILT signaling are as follows: ① bind to ILT-2 expressed on T cells, NK cells and B cells, inhibit the cytotoxicity of T cells and NK cells, and inhibit the proliferation and antibody secretion of B cells, etc.; ② bind to ILT-2 and ILT-4 expressed on dendritic cells (DC), inhibit the maturation and differentiation of DC cells, and induce the generation of tolerant DC cells; (3) bind to ILT-2 and ILT-4 expressed on myeloid-derived suppressor cells (MDSC) and macrophages (Mφ) to induce the differentiation of pro-inflammatory and anti-tumor M1 macrophages into M2 macrophages. Therefore, HLA-G plays an important role in the development and progression of tumors and other diseases. A number of HLA-G-targeted immunotherapy for solid cancers have been entered phase I clinical trials.

HLA-G binds to receptors ILT-2 and ILT-4 and has molecular structure specificity. The α3 domain of HLA-G extracellular region is the site where receptors ILT-2 and ILT-4 bind to HLA-G. ILT-2 only binds to the HLA-G/β₂m complex, while ILT-4 can not only bind to HLA-G/β₂m, but also bind to free HLA-G molecules that do not contain (β₂m. Due to differences in expression mechanisms and molecular structures of HLA-G1, -G2, -G3, -G4, -G5, -G6 and -G7 isoforms, ILT-2 can bind to HLA-G1 and HLA-G5, while ILT-4 can bind to HLA-G1, HLA-G2, HLA-G5 and HLA-G6 isoforms. For example, the extracellular regions of HLA-G3, -G4, and HLA-G7 isoforms contain no α3 domain and cannot bind to ILT-2 and ILT-4. Different HLA-G isoforms can exert specific immunological effects in pathophysiological processes. Under different pathological conditions, especially in tumor tissue cells, there is extensive heterogeneity in the expression of HLA-G isoforms, and the expressions of different HLA-G isoforms have specific clinical significance. Therefore, analyzing the expression of specific HLA-G isoforms and the expression profiles of different HLA-G isoforms is of great significance for elucidating the biological functions and clinical significance of specific HLA-G isoform.

Up to date, antibodies used for immunohistochemistry and immunoblotting of HLA-G molecule include 4H84, MEM-G1 and MEM-G2. Among them, the antibody 4H84 has a recognition site located in the extracellular region α1 domain for all α1 domain containing HLA-G isoforms. It can detect the seven HLA-G isoforms (HLA-G1, HLA-G2, HLA-G3, HLA-G4, HLA-G5, HLA-G6 and HLA-G7) that are currently known to contain α1 domains. Antibodies MEM-G1 and MEM-G2 are obtained from mice immunized with the full-length HLA-G heavy chain. The specific recognition sites cannot be predicted. Theoretically, the two antibodies are similar to the antibody 4H84 and can recognize the aforementioned HLA-G isoforms. However, the aforementioned antibodies 4H84, MEM-G1 and MEM-G2 have limitations in their detection specificity, and there are cross-reactions used for immunohistochemistry and immunoblotting, resulting in false positives. Meanwhile, the above three antibodies cannot be used for flow cytometry.

Therefore, in the context of targeted immunotherapy and precision medicine, the existing antibodies for detecting HLA-G molecule are not satisfactory, and there is an urgent need to develop monoclonal antibodies against HLA-G molecule with higher specificity and higher affinity.

### SUMMARY OF THE INVENTION

In view of the aforementioned shortcomings of existing HLA-G antibodies, an object of the present invention is to provide a specific monoclonal antibody YWHG-1 against HLA-G molecules, a nucleotide sequence encoding the antibody YWHG-1 and the encoded amino acid sequence thereof; and use of the YWHG-1 antibody for immunohistochemistry, immunoblotting, flow cytometry and other assays.

In the present invention, amino acid residues which are common to seven known HLA-G isoforms (HLA-G1, HLA-G2, HLA-G3, HLA-G4, HLA-G5, HLA-G6 and HLA-G7) and are located at positions 72-91 in the heavy chain α1 domain of the HLA-G molecule is used as an immunogen. The immunogen amino acid sequence is QTDRMNLQTLRGYYNQSEAS as shown in SEQ ID No. 19. The peptide (QTDRMNLQTLRGYYNQSEAS) is located in the heavy chain α1 domain of the HLA-G molecule (the area shown in the dashed box in FIG. 1), and based on the peptide fragment, the specific monoclonal antibody YWHG-1 against HLA-G molecules is prepared.

According to the inventor's research results, the present invention provides a monoclonal antibody YWHG-1 against HLA-G molecule, which at least includes one or more of light chain hypervariable regions CDR1, CDR2 and CDR3, or/and one or more of heavy chain hypervariable regions CDR1, CDR2 and CDR3;

An antibody light chain amino acid sequence of the monoclonal antibody YWHG-1 is as shown in SEQ ID No. 1 or an amino acid sequence with equivalent functions to the sequence shown in SEQID No. 1 formed by substituting, deleting or adding one or more amino acids; an amino acid sequence of the antibody light chain hypervariable region CDR1 is the sequence QSFVHSNGNIY shown in SEQ ID No.2 or an amino acid sequence with equivalent functions to the sequence shown in SEQID No.2 formed by substituting, deleting or adding one or more amino acids; an amino acid sequence of the antibody light chain hypervariable region CDR2 is the sequence KVS shown in SEQ ID No.3 or an amino acid sequence with equivalent functions to the sequence shown in SEQID No.3 formed by substituting, deleting or adding one or more amino acids; an amino acid sequence of the antibody light chain hypervariable region CDR3 is the sequence FQGSHVPPT shown in SEQ ID No.4 or an amino acid sequence with equivalent functions to the sequence shown in SEQID No.4 formed by substituting, deleting or adding one or more amino acids;
An antibody heavy chain amino acid sequence of the monoclonal antibody YWHG-1 is as shown in SEQ ID No.5 or an amino acid sequence with equivalent functions to the sequence shown in SEQID No.5 formed by substituting, deleting or adding one or more amino acids; an amino acid sequence of the antibody heavy chain hypervariable region CDR1 is the sequence GYIFTSYW shown in SEQ ID No.6 or an amino acid sequence with equivalent functions to the sequence shown in SEQID No.6 formed by substituting, deleting or adding one or more amino acids; an amino acid sequence of the antibody heavy chain hypervariable region CDR2 is the sequence IYPSDSYT shown in SEQ ID No.7 or an amino acid sequence with equivalent functions to the sequence shown in SEQID No.7 formed by substituting, deleting or adding one or more amino acids; an amino acid sequence of the antibody heavy chain hypervariable region CDR3 is the sequence TRFGYPFDY shown in SEQ ID No.8 or an amino acid sequence with equivalent functions to the sequence shown in SEQID No.8 formed by substituting, deleting or adding one or more amino acids.

Further, the monoclonal antibody YWHG-1 includes a light chain framework region and a heavy chain framework region; wherein, the light chain framework region (FR) includes one or more of light chain FR1, FR2, FR3 and FR4, and an amino acid sequence of the light chain FR1 is the sequence DIVITQDELSLTVSLGDQASISCRTS shown in SEQ ID No.9 or an amino acid sequence with equivalent functions to the sequence shown in SEQID No.9 formed by substituting, deleting or adding one or more amino acids; an amino acid sequence of the light chain FR2 is the sequence LEWFLQKPGQSPKLLIY shown in SEQ ID No. 10 or an amino acid sequence with equivalent functions to the sequence shown in SEQID No. 10 formed by substituting, deleting or adding one or more amino acids; an amino acid sequence of the light chain FR3 is the sequence SRFSGVPDRFSGSGSGTDFTLKISRVEAEDLGIYYC shown in SEQ ID No.11 or an amino acid sequence with equivalent functions to the sequence shown in SEQID No. 11 formed by substituting, deleting or adding one or more amino acids; an amino acid sequence of the light chain FR4 is the sequence FGGGTKLEIK shown in SEQ ID No. 12 or an amino acid sequence with equivalent functions to the sequence shown in SEQID No. 12 formed by substituting, deleting or adding one or more amino acids; the heavy chain framework region includes one or more of heavy chains FR1, FR2, FR3 and FR4, wherein, an amino acid sequence of the heavy chain FR1 is the sequence QLQESGTVLVRPGASVKLSCKAS shown in SEQ ID No. 13 or an amino acid sequence with equivalent functions to the sequence shown in SEQID No. 13 formed by substituting, deleting or adding one or more amino acids; an amino acid sequence of the heavy chain FR2 is the sequence INWVKQRPGQGLEWIGN shown in SEQ ID No. 14 or an amino acid sequence with equivalent functions to the sequence shown in SEQID No. 14 formed by substituting, deleting or adding one or more amino acids; an amino acid sequence of the heavy chain FR3 is the sequence NFNQKFEDKATLTVDTSSSTAYMQFSSPTSEDSAVYYC shown in SEQ ID No. 15 or an amino acid sequence with equivalent functions to the sequence shown in SEQID No. 15 formed by substituting, deleting or adding one or more amino acids; an amino acid sequence of the heavy chain FR4 is the sequence WGQGTTLTVSS shown in SEQ ID No. 16 or an amino acid sequence with equivalent functions to the sequence shown in SEQID No. 16 formed by substituting, deleting or adding one or more amino acids.

Further, a nucleotide sequence encoding the light chain in the monoclonal antibody YWHG-1 is the sequence shown in SEQ ID No. 17 or a nucleotide sequence with equivalent functions to the sequence shown in SEQID No. 17 formed by substituting, deleting or adding one or more nucleotides; a nucleotide sequence encoding the heavy chain in the monoclonal antibody YWHG-1 is the sequence shown in SEQ ID No. 18 or a nucleotide sequence with equivalent functions to the sequence shown in SEQID No. 18 formed by substituting, deleting or adding one or more nucleotides.

According to one aspect of the present invention, a preferred monoclonal antibody YWHG-1 against HLA-G molecules is provided. The monoclonal antibody YWHG-1 is produced by the hybridoma deposited under CCTCC NO: 202120, the depository institution is China Center for Type Culture Collection, located at Wuhan University, Wuhan, Hubei Province, P.R. China (postal code: 430072), and the deposit time is on 11^{th} August, 2021.

The present invention further provides use of the antibody YWHG-1 against HLA-G molecule for immunohistochemistry, immunoblotting, flow cytometry and other assays for HLA-G isoform molecules.

In order to have a clearer understanding of the inventive concepts and technical solutions herein, the present application will be further described below in conjunction with specific embodiments and drawings. The technical solutions in the embodiments are only preferred embodiments and should not be construed as limiting the scope of the present application. For those skilled in the art, several improvements and adjustments can be made without departing from the technical principles of the present application, and these improvements and adjustments should be considered to fall within the scope of protection of the present application.

Unless otherwise defined, all technical terms used herein shall have the same meanings as commonly understood by those of ordinary skill in the art. Abbreviations for amino acid residues are the standard 3-letter and/or 1-letter codes to stand for the 20 commonly used amino acids used in the art.

For the light chain hypervariable region or heavy chain hypervariable region mentioned in the present invention, the "hypervariable region" is also called a complementarity determining region (CDR).

The "sequence" mentioned herein may refer to a sequence containing certain biologically equivalent amino acids or "conservative substitutions", and "other sequence" may include functionally non-equivalent amino acids or "non-conservative substitutions", which are genetically modified to improve the properties of CDRs or CDR-containing antibodies. Without substantially affecting the antibody activity, those skilled in the art can perform operation on the sequences in the present application, i.e., substituting, adding and/or deleting one or more (for example, 1, 2, 3, 4, 5, 6 , 7, 8, 9 or 10 or more) amino acids to obtain variants of the antibody or functional fragment sequence thereof. They should be considered to be included in the scope of protection of the present application. For example, amino acids with similar properties are substituted in a variable region. The sequence of the variant mentioned in the present invention may be at least 95%, 96%, 97%, 98% or 99% identical to its source sequence. Sequence identity can be measured using sequence analysis software. For example, a computer program BLAST with default parameters, especially BLASTP or TBLASTN. The sequences of a variety of amino acids are detailed in the Sequence Listing.

### BRIEF DESCRIPTION OF THE DRAWINGS

FIG. 1 shows a structural model diagram of 7 different HLA-G isoforms and positions of immunogen peptide fragments.
FIG.2 shows isotyping of heavy chain and light chain of the antibody YWHG-1.
FIG.3 shows the antibody purity detection by SDS-PAGE of the YWHG-1.
FIG.4 shows measurement of the affinity constant by the ELISA method with the antibody YWHG-1.
FIG.5 shows detection of HLA-G1~HLA-G7 molecules by immunoblotting with the antibody YWHG-1.
FIG.6 shows detection of intracellular HLA-G1-HLA-G7 molecules by flow cytometry with the antibody YWHG-1.
FIG.7 shows detection of the expression of HLA-G molecules in gastric cancer tissues by immunoblotting with the antibody YWHG-1.
FIG.8 shows the use of the antibody YWHG-1 for immunohistochemical detection of HLA-G molecule expression in gastric cancer tissues.

### DETAILED DESCRIPTION

### 1. Preparation of monoclonal antibody YWHG-1against HLA-G molecule

### ① Synthesis of immunogen peptide

The immunogen peptide located at the positions 72-91 of the amino acid sequence in the heavy chain α1 domain of the HLA-G molecule was synthesized, with SEQ No. 19 QTDRMNLQTLRGYYNQSEAS.

### ② Mouse immunization

Four SPF grade BALB/c female mice were initially immunized at 60 µg/mouse, received the first booster immunization at 30µg/mouse, the second booster immunization at 30 µg/mouse, and the third booster immunization at 30µg/mouse. Blood was taken from the orbit to measure the serum titer. The plate was coated with "SEQ No. 19 QTDRMNLQTLRGYYNQSEAS", and then the titer of the immunized mice was measured by ELISA. The plate was coated with "SEQ No. 19 QTDRMNLQTLRGYYNQSEAS" overnight at 2 µg/ml; blocked with 2% milk at 37°C for 2h; serum was diluted gradiently by 2-fold starting from 200-fold; the blank control was PBS and the negative control was negative serum diluted by 200-fold. Before fusion, mice were shocked with 50 µg of "SEQ No. 19 QTDRMNLQTLRGYYNQSEAS" immunogen. In the fusion experiment, mouse spleen cells and SP2/0 cells were taken and fused using the PEG method. After the fusion, the cells were screened and cultured in semi-solid medium (containing HAT).

### ③ Hybridoma screening

The hybidoma (10 plates × 93 cells) were picked and cultured in a 96-well culture plate (previously plated with thymocytes, 100µL/well). The plate was coated with "SEQ No. 19 QTDRMNLQTLRGYYNQSEAS", and the "SEQ No. 19 QTDRMNLQTLRGYYNQSEAS" was diluted with the coating solution to a final concentration of 2µg/ml, 100µL/well, 4°C, overnight; then washed with the washing solution for 3 times, and blocked with 2% milk blocking solution, 200 µL/well, incubated at 37°C incubator for 2 hours; then washed 3 times with the washing solution. The primary antibody (cell culture supernatant), negative control (SP2/0 culture supernatant), blank control (PBS), and positive control (1000-fold dilution of positive serum in PBS) were added at 100µL/well, and incubated in a 37°C incubator for 1 hour; then washed 3 times with the washing solution. The secondary antibody diluted by 20,000-fold with PBS was added at 100 µL/well, and incubated in a 37°C incubator for 1 hour; taken out and washed 3 times with the washing solution. Then color development was performed with the developing solution at 100 µL/well for about 5 minutes. Then 50 µL of stop solution was added, and the absorbance was measured at dual wavelengths (450, 630). The selected clones were screened using ELISA for the first time to obtain a positive hybridoma cell line. The positive cell line was plated again with "SEQ No. 19 QTDRMNLQTLRGYYNQSEAS", and the ELISA method was used for a second screening to obtain a positive hybridoma cell line. After multiple screenings, a monoclonal antibody against the SEQ No. 19 QTDRMNLQTLRGYYNQSEAS fragment was obtained, which was named YWHG-1 (deposit number: CCTCC NO: 202120).

### ④ Isotyping of monoclonal antibody YWHG-1 against HLA-G molecules

The isotype of 10 screened positive cell lines was identified. The coated antibody was diluted with 100 mM PBS (pH7.4) to 0.5 µg/ml, 0.1 ml was added to each well, 4°C, overnight; washed twice with PBS-T, then 200 µL of blocking solution was added to each well, and incubated at 37°C for 2 hours; washed 3 times with PBS-T, then 100 µL of hybridoma supernatant was added to each well, and incubated at 37°C for 1 hour; washed 3 times with PBS-T, then 0.1 ml of HRP-labeled antibody diluted with the blocking solution 1: 10000 (κ, λ) or 1:20000 (others) was added to the appropriate wells, and incubated at 37°C for 1 hour; washed 3 times with PBS-T; 50 µL of the substrate solution was added to each well, and the absorbance was measured at dual wavelengths (450, 630). Moreover, The antibody isotype was identified using Thermo's Pierce Rapid Isotyping Kits (Catalog #26178), to determine that the isotype of the monoclonal antibody produced by the cell line was heavy chain IgG1 type and light chain kappa (κ) type (FIG.2).

### ⑤Antibody purification

Sample pretreatment: the sample was diluted with the corresponding coupling buffer in 1:3, and centrifuged at 12,000 rpm and 4°C for 10 min, and filtered with a 0.22 µm filter membrane to remove fats, cell debris, and small particulate matters. Equilibrate: the column was equilibrated with the corresponding coupling buffer at 10-fold column volume, maintaining the flow rate at 1 ml/min. Sample loading: The sample was injected into the upper port of the column, and the effluent was collected, keeping the flow rate at 1 ml/min. Washing: the coupling buffer at 5-fold column volume passed through the column, keeping the flow rate at 1 ml/min. Elution: the antibody was eluted using the elution buffer at 5-fold column volume, and collected into the above EP tubes, keeping the flow rate at 1 ml/min. The pH was adjusted to 7.0 with 1 M Tris-Hcl buffer. Equilibrate: the column was equilibrated with the coupling buffer at 10-fold column volume to pH 7.0, maintaining the flow rate at 1 ml/min. Dialysis: the antibody was dialyzed overnight using 0.01 M PBS buffer, which was changed 3 times.

### ⑥ Detection of antibody purity by SDS-PAGE

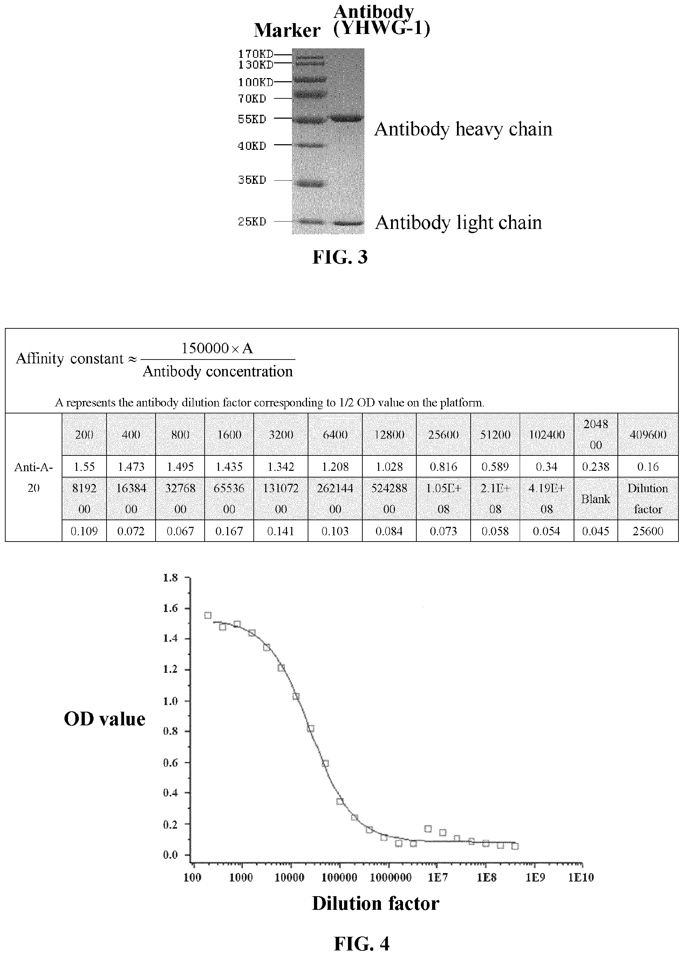

The SDS-PAGE gel was prepared, with the concentration of the separating gel of 12%. Sample preparation: the loading buffer was added to the sample, and boiled in boiling water for 10 minutes. Sample loading: 10 µL per well. Gel electrophoresis: 80V for stacking gel, 30 min; 120V for separating gel, 60 min. When the front of the bromophenol blue reached the bottom of the glass plate, the electrophoresis was stopped and the gel was taken out. Staining and destaining: the gel was immersed in Coomassie Brilliant Blue staining solution and slowly shaken on a shaker for more than 30 minutes (the staining time should be adjusted appropriately according to the gel thickness). The gel was taken out and rinsed in water several times, then Coomassie Brilliant Blue destaining solution was added again and shaken. The gel was destained for 1 hour until the bands are roughly visible. To destain completely, the destaining solution should be changed for 2-3 times while shaking for more than 24 hours. After destaining, the gel could be scanned and recorded by the ECL gel imaging system. The purity of the purified antibody YWHG-1 was higher than 90% (FIG.3).

### ⑦ Measurement of affinity constant of the antibody by ELISA

The antigen (SEQ No. 19 QTDRMNLQTLRGYYNQSEAS) was diluted with coating solution to a final concentration of 2 µg/ml, 10 µL/well, overnight at 4°C; then washed twice with the washing solution; blocked with the blocking solution at 200 µL/well, incubated at 37°C for 2 hours; then washed once with the washing solution. The purified antibody was diluted gradiently with PBS by 2-fold starting from 200-fold, using PBS as the blank control, both 100 µL/well, incubated at 37°C for 1 hour; and then washed 3 times with the washing solution. The secondary antibody diluted by 20,000-fold with PBS was added at 100 µL/well, and incubated in a 37°C incubator for 1 hour; taken out and washed 3 times with the washing solution. Then color development was performed with the developing solution at 100 µL/well for 5-15 minutes. Then 50 µL of stop solution was added, and the absorbance was measured at dual wavelengths (450, 630), and a plot was drawn for analysis. According to the Logistic fitting equation, the affinity constant was calculated as: 150000×A/antibody concentration (A: the antibody dilution factor corresponding to 1/2 OD value; dilution factor=25600; concentration of the antibody YWHG-1 =1.9 mg/mL). The calculated affinity constant of the antibody YWHG-1 was 2.02×10⁹ L/mol (FIG.4).

### ⑧ The specificity of HLA-G isoform recognition detected by immunoblotting

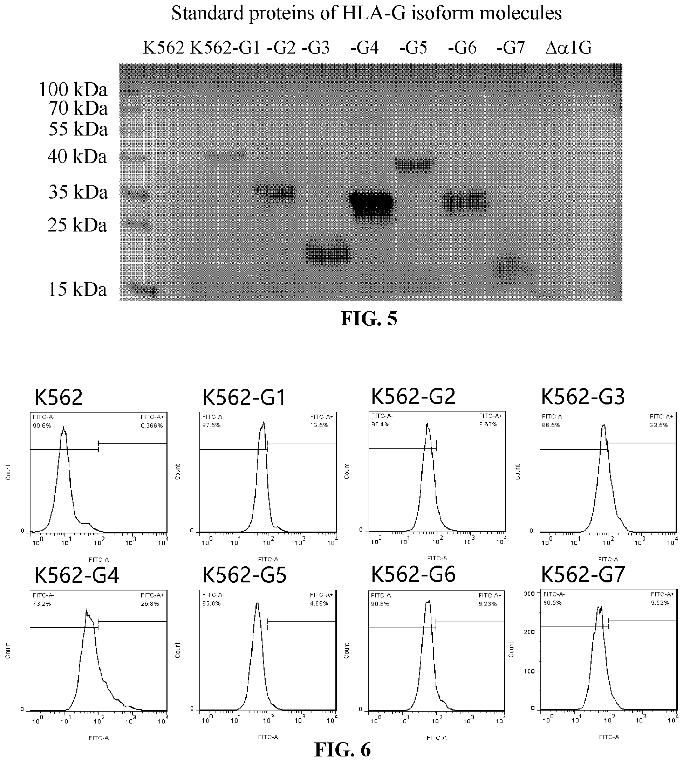

After SDS-PAGE electrophoresis and transferred to PVDF membrane, the HLA-G isoform standard proteins HLA-G1, HLA-G2, HLA-G3, HLA-G4, HLA-G5, HLA-G6, HLA-G7 and HLA-G molecular standard proteins lacking α1 domain were blocked with 5% skimmed milk powder at room temperature for 4 hours, and washed with 0.2% TBS (Tewen-20 PBS). The antibody (YWHG-1, 1.0 µg/ml) was added, incubated at 4°C overnight, and washed; then HRP-labeled rabbit anti-mouse IgG antibody was added and incubated at room temperature for 30 minutes, washed, and incubated with Dako REAL^{™} EnVision^{™} testing system (DAKO) for 1-3 minutes. The results showed that the antibody (YWHG-1) could specifically recognize α1 domain containing HLA-G isoforms (HLA-G1, HLA-G2, HLA-G3, HLA-G4, HLA-G5, HLA-G6, HLA-G7) (FIG.5).

### 2. Examples

### Example 2.1 Detection of HLA-G1~HLA-G7 molecules by flow cytometry with the antibody YWHG-1

The cell cultures expressing the K562 (blank control), K562-HLA-G1, K562-HLA-G2, K562-HLA-G3, K562-HLA-G4, K562-HLA-G5, K562-HLA-G6 and K562-HLA-G7 in the logarithmic growth phase were used. The detection was conducted by intracellular flow cytometry.

Detection of intracellular molecule expression by flow cytometry: The cell cultures of K562, K562-HLA-G1, K562-HLA-G2, K562-HLA-G3, K562-HLA-G4, K562-HLA-G5, K562-HLA-G6, K562-HLA-G7 were collected in test tubes, centrifuged and washed twice with 2% BSA/PBS (250 g), then 250 µL of cell membrane-breaking agent (BD Cytofix/Cytoperm^{™}) was added, fully mixed, and placed in the refrigerator at 4°C for 20 minutes. Then 1 ml of BD Perm/Wash^{™} was added to centrifuge and wash twice (250 g). After washing, K562, K562-HLA-G1, K562-HLA-G2, K562-HLA-G3, K562-HLA-G4, K562-HLA-G5, K562-HLA-G6 and K562-HLA-G7 cells were resuspended with 100 µL of BD Perm/Wash^{™}, and then 1 µL (1.0 mg/ml) of FITC-labeled purified antibody (YWHG-1) was added, incubated at 4°C for 30 minutes, and centrifuged and washed 3 times with 1 ml of 1× BD Perm/Wash^{™} (250g ); the cells were resuspended to 300 µL of cell suspension with 1× BD Perm/Wash^{™} for flow cytometry. The results showed that the antibody (YWHG-1) could specifically recognize the HLA-G1, HLA-G2, HLA-G3, HLA-G4, HLA-G5, HLA-G6, and HLA-G7 molecules (FIG. 6).

### Example 2.2 Detection of expression of HLA-G molecules in gastric cancer tissues by immunoblotting with the antibody (YWHG-1)

Preparation of single cell suspensions of frozen gastric cancer tissues from 62 patients: mechanical separation method and enzymatic digestion method were adopted. The steps were as follows: take out the sample, equilibrate to room temperature, wash with HBSS buffer for three times, and then cut into tissue fragments with a size of 5-6 mm. Wash the tissue fragments with HBSS buffer for three times, then digest with type IV collagenase (1 mg/ml) and hyaluronidase (10 ng/ml) at 37°C in 2% fetal bovine serum (FBS) HBSS buffer for 4 hours. After digestion, filter the cells using a 70 µm cell filter to obtain a cell suspension. After centrifugation, use a tissue cell lysis buffer to lyse at 4°C for 2 hours, centrifuge at 12,000 rpm for 30 minutes, and collect the lysis supernatant for later use.

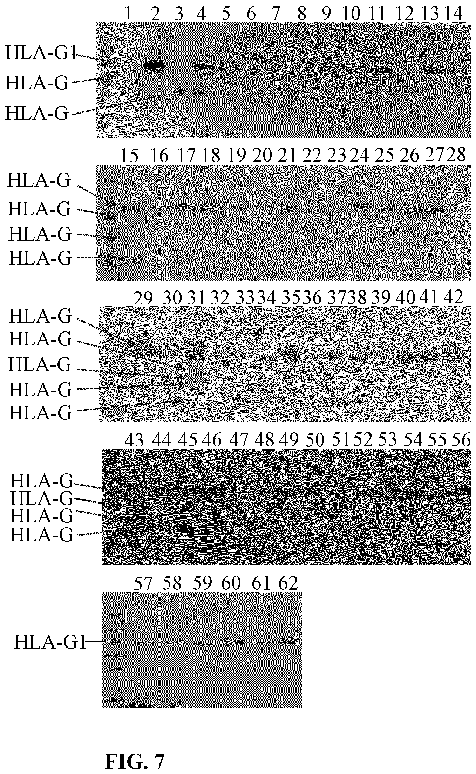

Carry out denaturing PAGE of the lysed supernatant of gastric cancer tissues of 62 patients; after semi-dry transferred to PVDF membrane, block with 2% albumin/PBS at room temperature for 4 hours, and wash with 0.2% TBS (Tewen-20 PBS). Add the antibody YWHG-1 to detect the expression of HLA-G molecule. Incubate overnight at 4°C, wash; add HRP-labeled rabbit anti-mouse IgG antibody, incubate at room temperature for 30 minutes, wash, and incubate with Dako REAL^{™} EnVision^{™} detection system (DAKO) for 1-3 minutes. The results showed that the antibody YWHG-1 could specifically detect the expression of HLA-G molecules in colorectal cancer tissues (FIG.7).

### Example 2.3 Detection of expression of HLA-G molecules in gastric cancer tissues by immunohistochemstry with the antibody YWHG-1

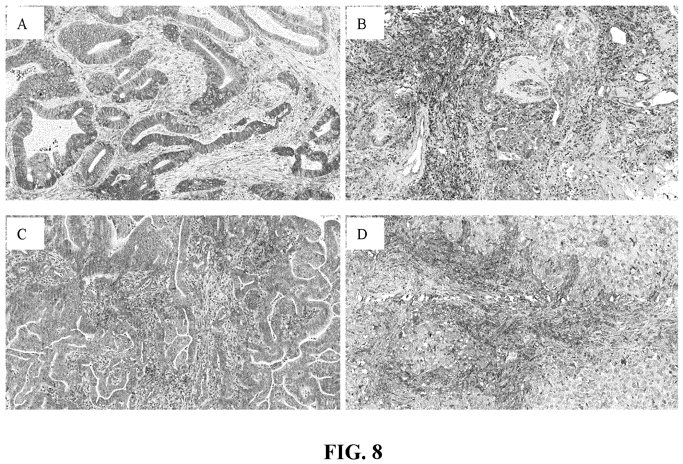

The gastric cancer tissues were fixed in 10%-12% formalin and embedded in paraffin. The conventional process for making tissue sections was carried out, such as baking, dewaxing, hydration and antigen retrieval. On the tissues, 1% BSA was added dropwise to cover the tissue and tissue edges by 2 mm, and incubated at room temperature for 10 minutes and then blocked. The antibody YWHG-1 (1 mg/mL, 1:500 dilution) was added dropwise and kept in a wet box of the refrigerator at 4°C overnight (16-20 h). After washing with TBS buffer, the secondary antibody (the antibody goat anti-mouse diluted with TBS in a ratio of 1 :300) was added, incubated in a 37°C incubator for 30 minutes; washed with TBS buffer, and the developing agent DAB working solution was added; when the tissue color development was completed, the slides was rinsed in running water for 5 minutes and soaked in distilled water for 5 minutes. After HE counterstaining, dehydration, transparency, and sealing, the tissue sections were observed under optical microscope. Brown-colored cells indicated positive expression of HLA-G molecule in gastric cancer cells. The expression intensity of HLA-G molecules could be judged according to the depth of the brown-colored cells. FIG.8 showed high expression of HLA-G molecules in the gastric cancer tissue samples (A), (B), (C) and (D) (FIG.8).

## Claims

1. A monoclonal antibody YWHG-1 against HLA-G molecule, wherein the monoclonal antibody YWHG-1 is produced by a hybridoma deposited under CCTCC NO: 202120; the depository institution is China Center for Type Culture Collection, and the deposit time is August 11, 2021.

2. A monoclonal antibody YWHG-1 against HLA-G molecule, wherein the monoclonal antibody YWHG-1 at least comprises one or more of light chain hypervariable regions CDR1, CDR2 and CDR3, and/or one or more of heavy chain hypervariable regions CDR1, CDR2 and CDR3;
an antibody light chain amino acid sequence of the monoclonal antibody YWHG-1 is as shown in SEQ ID No.1 or an amino acid sequence with equivalent functions to the sequence shown in SEQID No.1 formed by substituting, deleting or adding one or more amino acids; an amino acid sequence of the antibody light chain hypervariable region CDR1 is the sequence QSFVHSNGNIY shown in SEQ ID No.2 or an amino acid sequence with equivalent functions to the sequence shown in SEQID No.2 formed by substituting, deleting or adding one or more amino acids; an amino acid sequence of the antibody light chain hypervariable region CDR2 is the sequence KVS shown in SEQ ID No.3 or an amino acid sequence with equivalent functions to the sequence shown in SEQID No.3 formed by substituting, deleting or adding one or more amino acids; an amino acid sequence of the antibody light chain hypervariable region CDR3 is the sequence FQGSHVPPT shown in SEQ ID No.4 or an amino acid sequence with equivalent functions to the sequence shown in SEQID No.4 formed by substituting, deleting or adding one or more amino acids;
an antibody heavy chain amino acid sequence of the monoclonal antibody YWHG-1 is as shown in SEQ ID No.5 or an amino acid sequence with equivalent functions to the sequence shown in SEQID No.5 formed by substituting, deleting or adding one or more amino acids; an amino acid sequence of the antibody heavy chain hypervariable region CDR1 is the sequence GYIFTSYW shown in SEQ ID No.6 or an amino acid sequence with equivalent functions to the sequence shown in SEQID No.6 formed by substituting, deleting or adding one or more amino acids; an amino acid sequence of the antibody heavy chain hypervariable region CDR2 is the sequence IYPSDSYT shown in SEQ ID No.7 or an amino acid sequence with equivalent functions to the sequence shown in SEQID No.7 formed by substituting, deleting or adding one or more amino acids; an amino acid sequence of the antibody heavy chain hypervariable region CDR3 is the sequence TRFGYPFDY shown in SEQ ID No.8 or an amino acid sequence with equivalent functions to the sequence shown in SEQID No.8 formed by substituting, deleting or adding one or more amino acids.

3. The monoclonal antibody YWHG-1 against HLA-G molecule according to claim 2, wherein the monoclonal antibody YWHG-1 further comprises a light chain framework region (FR) and a heavy chain framework region; the light chain framework region comprises one or more of light chain FR1, FR2, FR3 and FR4, and an amino acid sequence of the light chain FR1 is the sequence DIVITQDELSLTVSLGDQASISCRTS shown in SEQ ID No.9 or an amino acid sequence with equivalent functions to the sequence shown in SEQID No.9 formed by substituting, deleting or adding one or more amino acids; an amino acid sequence of the light chain FR2 is the sequence LEWFLQKPGQSPKLLIY shown in SEQ ID No. 10 or an amino acid sequence with equivalent functions to the sequence shown in SEQID No. 10 formed by substituting, deleting or adding one or more amino acids; an amino acid sequence of the light chain FR3 is the sequence SRFSGVPDRFSGSGSGTDFTLKISRVEAEDLGIYYC shown in SEQ ID No.11 or an amino acid sequence with equivalent functions to the sequence shown in SEQID No. 11 formed by substituting, deleting or adding one or more amino acids; an amino acid sequence of the light chain FR4 is the sequence FGGGTKLEIK shown in SEQ ID No. 12 or an amino acid sequence with equivalent functions to the sequence shown in SEQID No. 12 formed by substituting, deleting or adding one or more amino acids;
the heavy chain framework region comprises one or more of heavy chain FR1, FR2, FR3 and FR4, and an amino acid sequence of the heavy chain FR1 is the sequence QLQESGTVLVRPGASVKLSCKAS shown in SEQ ID No. 13 or an amino acid sequence with equivalent functions to the sequence shown in SEQID No. 13 formed by substituting, deleting or adding one or more amino acids; an amino acid sequence of the heavy chain FR2 is the sequence INWVKQRPGQGLEWIGN shown in SEQ ID No. 14 or an amino acid sequence with equivalent functions to the sequence shown in SEQID No. 14 formed by substituting, deleting or adding one or more amino acids; an amino acid sequence of the heavy chain FR3 is the sequence NFNQKFEDKATLTVDTSSSTAYMQFSSPTSEDSAVYYC shown in SEQ ID No. 15 or an amino acid sequence with equivalent functions to the sequence shown in SEQID No. 15 formed by substituting, deleting or adding one or more amino acids; an amino acid sequence of the heavy chain FR4 is the sequence WGQGTTLTVSS shown in SEQ ID No. 16 or an amino acid sequence with equivalent functions to the sequence shown in SEQID No. 16 formed by substituting, deleting or adding one or more amino acids.

4. The monoclonal antibody YWHG-1 against HLA-G molecules according to claim 2, wherein a nucleotide sequence encoding the light chain variable region in the monoclonal antibody YWHG-1 is the sequence shown in SEQ ID No. 17 or a nucleotide sequence with equivalent functions to the sequence shown in SEQID No. 17 formed by substituting, deleting or adding one or more nucleotides; a nucleotide sequence encoding the heavy chain variable region in the monoclonal antibody YWHG-1 is the sequence shown in SEQ ID No. 18 or a nucleotide sequence with equivalent functions to the sequence shown in SEQID No. 18 formed by substituting, deleting or adding one or more nucleotides.

5. Use of the monoclonal antibody YWHG-1 against HLA-G molecule of any one of claims 1 to 4 for immunohistochemistry, immunoblotting and intracellular flow cytometry.
